# EUROPEAN PATENT APPLICATION

(11) **EP 2 626 373 A1**
(43) Date of publication of application: **14.08.2013**
(21) Application number: 12154485.2
(22) Date of filing: 08.02.2012
(51) Int. Cl.: C08B 37/00, C08L 5/00, C02F 5/10

(54) **Method for the manufacture of concentrated aqueous solutions of alkali metal salt of carboxymethyl fructan**

(71) Applicant: Dequest AG, 6300 Zug (CH)
(72) Inventor: Notté, Patrick Pierre, B-1300 Wavre (BE); Devaux, Albert Firmin, B-1435 Mon-Saint-Guibert (BE)
(74) Representative: Pronovem

(57) **Abstract**

The present invention is directed to a new method for the manufacture of aqueous solutions of alkali metal salt of carboxymethyl fructan. More specifically the present invention relates to a new method for the manufacture of aqueous solutions comprising at least 20% by weight of alkali metal salt of carboxymethyl fructan having a degree of carboxymethyl substitution of at least 1.2.

## Description

### Field of the Invention

The present invention is related to a new method for the manufacture of concentrated, homogeneous aqueous solutions of alkali metal salt of carboxymethyl fructan, with a degree of carboxymethyl substitution higher than 1, wherein said aqueous solutions comprise a reduced level of alkali metal halogenide salt.

### State of the Art

Fructans are oligo- and polysaccharides, which have a majority of anhydrofructose units. The fructans can have a polydisperse chain length distribution and can be linear or branched. The fructans have an average chain length (degree of polymerization; DP) of at least 3 to about 1000. Inulin is a fructan comprising (2-1)-B-D-fructofuranosyl residues with an α-D-glucopyranosyl unit at the reducing end. This fructan can be found as a reserve polysaccharide in various plants such as chicory, Jerusalem artichoke and dahlia.

Carboxymethylation is a known process for the derivation of mono-, oligo- and polysaccharides by which the primary and/or secondary alcohol groups are etherified with carboxymethyl groups. The derivatives thus obtained are (poly)-methylene carboxylates, which find use in many applications, especially in detergent formulations, in the paper industry and in oil winning.

Known examples of carboxymethylated products are carboxymethyl cellulose (CMC) as for example described in US 2,639,281, US 3,284,441 and CA 523,243

The prior art relating to carboxyalkyl inulins as such and methods for the manufacture thereof is fairly substantial and diverse. Carboxymethyl inulin salts have been known for a long time, see e.g. Czech patent No 90980 of 1958/1959. Chien, J. Immunology Methods 26 (1979) 39-46, pertains to the preparation of carboxymethyl inulin (CMI) thereby using a six-fold molar excess of sodium chloroacetate in diluted alkaline solution at relatively low temperature. The CMI so prepared has a very low degree of substitution and the method is cumbersome and not suitable for any economically viable undertaking.

EP 0733073 describes a method for the manufacture of carboxymethyl inulin by reacting, under alkaline conditions, inulin and sodium chloroacetate. The technology is, inter alia, subject to substantial viscosity difficulties which can lead to low conversions and undesirable by-products. Besides, working up of the reaction mixture consists in precipitating the reaction mixture at the end of the reaction with a suitable solvent, such as absolute methanol.

EP 1713831 describes a method for the manufacture of alkali metal salt of carboxyalkyl inulin comprising preparing an aqueous medium containing dispersed therein an alkali metal salt of halogenoalkylcarboxylate, adding to the carboxylate containing medium, under substantially neutral pH conditions, an inulin followed by heating this mixture to a temperature in the range of from 60°C to 90°C and proceeding with the reaction at alkaline conditions, pH 8-12, while simultaneously adding additional halogenoalkylcarboxylate and alkaline hydroxide. The method as claimed in EP 1713831 is not subject to viscosity problems and allows for the preparations of carboxyalkyl inulins with acceptable yields, purity and degrees of conversion having a degree of carboxyalkyl substitution (DS) above about 1.5 up to about 3. The alkali metal salt of the carboxyalkyl inulin, so formed, is generally diluted with water in order to have homogeneous CMI solutions in water.

Dependent of their degree of carboxymethyl substitution, only low carboxymethyl inulin-concentrations are stable in water at room temperature and below. At present aqueous solutions with carboxymethyl inulin concentrations of up to 15% by weight can be made for a degree of carboxymethyl substitution of 2.5 and of up to 25% by weight for a degree of carboxymethyl substitution of 1.5.

Carboxyalkyl inulin can also be recovered and purified with the aid of conventional methods well known in this domain of the technology. Nanofiltration followed by evaporation are used to remove most of the low molecular weight impurities like sodium chloride and glycolic/diglycolic acids. This technique allows to obtain higher carboxy alkyl inulin concentrates, above 25% by weight. Nevertheless, this technique is time and energy consuming, uneconomical and less adapted to actual and foreseeable commercial needs.

### Aims of the Invention

The present invention aims to provide a method for the manufacturing of aqueous solutions of alkali metal salt of carboxymethyl fructan, preferably inulin that does not present the drawbacks of the methods of the state of the art.

It is an aim of the present invention to provide an efficient and economical method for the manufacture of a homogeneous aqueous solution of alkali metal salt of carboxymethyl fructan, preferably inulin, the alkali metal salt of carboxymethyl fructan, preferably inulin, having a degree of carboxymethyl substitution of from above 1.2 up to 3.0, the aqueous solution having an alkali metal salt of carboxymethyl fructan, preferably inulin, concentration of at least 20% by weight and a alkali metal halogenide salt concentration of at most 10% by weight. Therefore, a further aim of the present invention is to obtain such concentrated solutions that are more easily and less costly manufactured and transported and that are stable at room temperature and below, i.e. in normal use and shipping conditions.

### Summary of the Invention

The present invention discloses a method for the manufacture of an aqueous solution of alkali metal salt of carboxymethyl fructan, preferably inulin, comprising the steps of:
a) reacting, in an aqueous medium, a fructan, preferably inulin, with an alkali metal salt of halogenomethylcarboxylate in the presence of an alkaline hydroxide at a temperature comprised between about 50° C and about 100°C to form a reaction medium;
b) cooling the reaction medium to a temperature lower than 40°C to form a slurry comprising an alkali metal halogenide precipitate;
c) separating the alkali metal halogenide precipitate from the slurry and collecting an aqueous solution comprising at least 20% by weight of alkali metal salt of carboxymethyl fructan, preferably inulin, with a degree of carboxymethyl substitution of at least 1.2.

Preferred embodiments of the present invention disclose one or more of the following features:
- step a) comprises the steps of:
   a.1) adding a fructan, preferably inulin, and an alkaline hydroxide to an aqueous medium while heating to a temperature between about 60°C and about 100°C, to yield a reaction medium comprising between about 25 % and about 70% by weight of fructan (inulin), expressed in relation to the amount of water, and characterized in that the pH is in the range of from about 8 to about 13;
   a.2) simultaneously gradually adding an alkali metal salt of a halogenomethyl carboxylate and an alkaline hydroxide to yield a molar ratio of alkali metal salt of a halogenomethylcarboxylate to the repetitive monosaccharide unit of fructan (inulin) of from about 1.0 to about 5.0 and a pH in the range of from about 8 to about 13;
   a.3) maintaining the reaction medium, at a temperature comprised between about 60°C and about 100°C and completing the carboxymethylation reaction;
- the aqueous medium of step a) is water or an aqueous dispersion comprising from about 25% to about 150% mole, expressed in relation to the molar amount of monosaccharide units in the fructan (inulin)(100%), of an alkali metal salt of a halogenomethylcarboxylate;
- the alkali metal salt of the halogenomethylcarboxylate of step a), wherein the halogen is selected from the group consisting of chlorine, bromine, and iodine and the alkaline ion is selected from the group consisting of sodium and potassium;
- the alkali metal salt of halogenomethylcarboxylate of step a) is the sodium salt of monochloroacetic acid or the potassium salt of a monochloroacetic acid or a mixture thereof;
- the aqueous solution obtained from step c) comprises between about 20% and about 50 % by weight and preferably between about 25% and about 40% by weight of an alkali metal salt of carboxymethyl fructan (inulin);
- the alkali metal salt of carboxymethyl fructan (inulin) of the aqueous solution obtained from step c) presents a degree of carboxymethyl substitution comprised between about 1.2 and about 3.0;
- the aqueous solution obtained from step c) comprises less than 10% by weight of an alkali metal halogenide salt;
- step a.1) and a.2) are performed while maintaining the temperature of the reaction medium comprised between about 70° C and about 90° C and the pH of in the range of from about 9.5 to about 11.5;
- step a.3) is performed while maintaining the temperature of the reaction medium comprised between about 70°C and about 90°C;
- the reaction medium of step a) of the present method is cooled down in step b) to a temperature comprised between about 15°C and about 30°C, preferably between about 15°C and about 25°C;
- step c) is performed by using conventional separation techniques, preferably by centrifugation techniques;
- the aqueous solution of an alkali metal salt of carboxymethyl fructan (inulin), preferably obtained according to the method of the present invention, comprises at least 20% weight of alkali metal salt of carboxymethyl fructan(inulin) wherein the alkali metal salt of carboxymethyl fructan (inulin) is characterized by a degree of carboxymethyl substitution of at least 1.2;
- the aqueous solution of an alkali metal salt of carboxymethyl fructan (inulin) preferably obtained according to the method of the present invention, comprises between about 20% and about 50% by weight of an alkali metal salt of carboxymethyl fructan, between about 5% and about 10% by weight of an alkali metal halogenide salt, between about 5% and about 15% by weight of an alkali metal glycolate salt and between about 1% and about 10% by weight of an alkali metal diglycolate salt;
- the aqueous solution of alkali metal salt of carboxymethyl fructan(inulin) preferably obtained according to the method of the present invention can be used as a scale inhibitor, as a dispersing agent and/or as a metallic cation sequestering agent.

### Detailed Description of the Invention

The method of the invention is highly versatile in that it provides direct access to concentrated aqueous solutions of alkali metal salt of carboxymethyl fructan (preferably inulin), the aqueous solutions being further characterized in having a low alkali metal halogenide content; the alkali metal salt of carboxymethyl fructan (inulin) being further characterized by a high degree of carboxymethyl substitution. The degree of carboxymethyl substitution defines the average number of hydroxyl functional groups per fructose unit of the fructan which have been substituted by a carboxymethyl group.

An important characteristic of the method of the present invention is that upon cooling the reaction medium of step a) in step b), i.e. after completion of the carboxymethylation reaction, there is a substantial selective precipitation of alkali metal halogenide salt meanwhile the alkali metal salt of carboxymethyl fructan (inulin) remains substantially dissolved in the aqueous medium at a concentration of at least 20% by weight. This substantial selective precipitation of alkali metal halogenide salt from an aqueous medium is contrary to what one skilled in the art would expect.

The method of the invention allows obtaining homogeneous solutions of alkali metal salt of carboxymethyl fructan (inulin), with a reduced level of an alkali metal halogenide salt, without the application of the conventionally used membrane filtration techniques, such as nanofiltration, where diluted solutions are required for performing the filtration and where concentration of the filtrate for example through distillation, is needed afterwards.

The method of the invention presents considerable economical benefits not only because of the efficient use of raw materials and energy, but also because of the reduced transportation cost (less solvent has to be transported).

The fructan, preferably inulin considered within the scope of the present invention is an oligosaccharide consisting of (2-1)-linked β-D-fructofuranosyl residues with an α-D-glucopyranosyl unit at the reducing end and which is preferably characterized by an average chain length of from 3 to 60, in particular of from 5 to 30 monosaccharide units. Modified fructans, suitable for use within the claimed technology, can be represented by levans, graminans and inulins with enzymatically increased chain length, fructan hydrolysis products (fructooligosaccharides) having shortened chains and fractionated products having a modified chain length. Fractionation of inulin's can be achieved, for example, by means of known technologies including low temperature crystallization (see WO 94/01849), column chromatography (see WO 94/12541), membrane filtration (see EP-A-0440074, EP-A-0627490) or selective precipitation with alcohol. Hydrolysis to yield shorter fructans can be carried out, for example, enzymatically (endo-insulase), chemically (water and acid) or by heterogeneous catalysis (acid ion-exchange resins). Reduced, oxidized, hydroxyalkylated and/or cross-linked fructans can also represent suitable starting materials. In actual practice preference is given to an inulin having an average DP ranging from about 10 to about 30.

The alkali metal salt of halogenomethylcarboxylate within the scope of the present invention is selected from the sodium- or potassium salt of monochloromethylcarboxylate, monobromomethyl c**arboxylate** or monoiodomethylcarboxylate and any combinations or mixtures thereof.
Preferably, within the scope of the present invention the alkali metal salt of halogenomethylcarboxylate is the sodium salt of monochloroacetic acid.

The alkaline hydroxide within the scope of the present invention is selected from sodium hydroxide or potassium hydroxide or mixtures, used at 100 % or as an aqueous solution. Preferably, the alkaline hydroxide within the scope of the present invention is used as a 50 % by weight in water.
Within the scope of the present invention:

- The aqueous medium at the start of step a) is selected from:
- water,
- an aqueous dispersion that comprises from about 25% to about 150% mole, preferably from about 70% to about 100% mole, expressed in relation to the molar amount of monosaccharide units in the fructan (inulin) (100%) of an alkali metal salt of halogenomethylcarboxylate; the dispersion being continuously stirred at a temperature between about 25°C and about 45°C,
- an aqueous dispersion that contains up to 35% by weight, in one preferred execution from about 10% to about 30% by weight, of the fructan (inulin) (expressed in relation to the aqueous medium) (the use of aqueous fructan (inulin) solutions as the aqueous medium can be in order depending upon the fructan (inulin) starting material, which can conveniently be made available as a solution),
- an aqueous dispersion that contains up to 35% by weight, in one preferred execution from about 10% to about 30% by weight of the fructan (inulin) (expressed in relation to the aqueous medium) and that comprises from about 25% to about 150% mole, preferably from about 70% to about 100% mole, expressed in relation to the molar amount of monosaccharide units in the fructan (inulin) (100%) of an alkali metal salt of halogenomethylcarboxylate; the dispersion being continuously stirred at a temperature comprised between about 25°C and about 45°C.

- The fructan (inulin) addition in step a.1) is performed while stirring and the temperature is gradually increased to a value comprised between about 70°C and about 90°C.

- The fructan (inulin) addition in step a.1) yields a slurry with an inulin concentration comprised between about 25% and about 70 % by weight, preferably between about 40% and about 60% by weight expressed in relation to the amount of water in the slurry.

- The slurry of step a.1) optionally comprises an amount of alkaline hydroxide comprised between about 50% and about 150% mole expressed in relation to the molar amount of alkali metal salt of halogenomethylcarboxylate. The alkaline hydroxide is preferably gradually added as an aqueous solution.

- The simultaneous gradual addition of alkali metal salt of halogenomethylcarboxylate and alkaline hydroxide in step a.2) is performed in a period comprised between about 60 minutes and about 180 minutes to yield a reaction mixture with a pH comprised between about 8 and about 12, preferably between about 9.5 and about 11.5, measured at the temperature of the reaction medium, which is comprised between about 70°C and about 90°C.

- The simultaneous gradual addition of alkali metal salt of halogenomethylcarboxylate and alkaline hydroxide in step a.2) is performed in such a way that the temperature of the reaction medium remains unchanged at the set value.

-The quantity of alkali metal salt of halogenomethylcarboxylate added in step a.2) is in relation to the quantity of fructan (inulin) present in the slurry of step a.1) and is such that the molar ratio of the total of alkali metal salt of halogenomethylcarboxylate to the repetitive monosaccharide units of fructan (inulin) is between about 1.0 and about 5.0, preferably between about 1.5 and about 4.5.

- The quantity of alkaline hydroxide added in step a.2) is equimolar to the total of metal salt of halogenomethylcarboxylate added to the reaction medium. Optionally an additional amount of alkaline hydroxide, up to 50% mole in relation to the molar amount of monosaccharide units of the fructan (inulin) is added.

- The quantity of alkaline hydroxide added in step a.2) is such that the pH of the reaction medium is between about 8 and about 12, preferably between about 9.5 to about 11.5 measured at the temperature of the reaction medium (between about 70°C and about 90°C).

- After all the reagents have been added, step a.3) is performed while stirring and maintaining the temperature of the reaction medium comprised between about 70°C and about 90°C for a period of time comprised between about 30 minutes and about 4 hours, that is till completion of the carboxymethylation reaction.

- The reaction medium of step a) comprises an alkali metal salt of carboxymethyl fructan (inulin), an alkali metal halogenide salt, an alkali metal glycolate salt and/or an alkali metal diglycolate salt.

- An alkali metal halogenide salt is formed through the reaction of fructan (inulin) and a metal salt of an halogenomethylcarboxylate in the presence of an alkaline hydroxide.

- The reaction medium of step a) of the present method is cooled down, while stirring, in step b) to a temperature comprised between about 15°C and about 25°C, causing the alkali metal halogenide salt, produced in step a), to precipitate.

- The alkali metal halogenide salt precipitate is separated in step c) of the present method, from the alkali metal salt of carboxymethyl fructan (inulin) comprising aqueous solution by using conventional separation techniques, preferably by centrifugation techniques.

- The alkali metal salt of carboxymethyl fructan (inulin) according to the invention and preferably obtained from the method of the present invention is characterized by a degree of carboxymethyl substitution between about 1.2 and about 3.0. The degree of carboxymethyl substitution may be easily determined/calculated by those skilled in the art of organic chemistry using techniques well know to the skilled person such as titration of the organic acid groups, by HPLC analysis and by ¹³C NMR.

- The aqueous solution obtained from step c) of the present method comprises from about 20% to about 50% by weight and preferably from about 25% to about 40% by weight of alkali metal salt of carboxymethyl fructan (inulin).

- The aqueous solution obtained from step c) of the present method further comprises between about 5% and about 10% by weight of an alkali metal halogenide salt, between about 5% and about 15% by weight of an alkali metal glycolate salt and between about 1% and about 10% by weight of an alkali metal diglycolate salt.

- The aqueous solution of alkali metal salt of carboxymethyl fructan (inulin) according to the invention and preferably obtained according to the method of the present invention is intended for being used in applications based on a predominantly aqueous medium.

- The aqueous solution of alkali metal salt of carboxymethyl fructan (inulin) according to the invention and preferably obtained according to the method of the present invention is intended for being used as a scale inhibitor, a dispersing agent and/or a metal cation sequestering agent. Examples of controllable metal ions include earth alkali metal ions, such as calcium, strontium, barium and magnesium and metal ions, such as iron, chromium, manganese, cobalt, nickel and copper. Effectively controlling the formation of inorganic deposits, in particular inhibiting the formation of the like deposits, including frequently calcium carbonate, manganese carbonate, barium sulphate and strontium sulphate, in water is well know.

### Examples

The following examples illustrate the invention; they are merely meant to exemplify the present invention but are not destined to limit or otherwise define the scope of the present invention.

### Example 1.

In a 1 liter round-bottom flask, 30.9 g of monochloroacetic acid sodium salt (SMCA) were dissolved into 113.6 g of water under stirring, while the reaction flask was heated up to a temperature of about 35-40°C. To this solution, 113.6 g of Inulin (Fibruline® instant from Cosucra-Warcoing) were added, under vigorous stirring. A viscous slurry was obtained. The temperature was gradually increased to 70°C. Subsequently a 50% by weight aqueous solution of sodium hydroxide was added to the slurry at an overall rate of 2.3 g per minute during 6 minutes. The reaction medium, thus obtained, has a temperature of about 75°C and a pH value above 10.2.

Then, a simultaneous addition of a 50% by weight aqueous solution of sodium hydroxide, on the one hand, and SMCA on the other hand was carried out in such a way as to maintain the reaction temperature in the range 74 to 78°C and the pH value above 10.2;
Therefore:
- the 50% by weight aqueous solution of sodium hydroxide was added at an overall rate of 1.7 g per minute during 128 minutes(or a total amount of 217.6 g);
- the solid SMCA was added in portions of at an overall rate of 2.3 g per minute during 121 minutes (or a total amount of 278.3 g).

After completion of the reactants addition, the dark slurry (total amount of 767.8g) was maintained at 75°C for a further period of 1 hour under stirring.

Then, the reaction mixture was cooled down, while stirring, to ambient temperature whereupon a precipitate was formed.

The precipitate was separated from the aqueous solution: 741.5g of the above slurry were poured into a centrifuge whereupon 131.6 g of precipitate and 609.9 g of aqueous solution were obtained.

The operating conditions of the solid isolation are as follows: The equipment used to isolate the solid fraction from the viscous liquid is a centrifuge Rousselet Robatel® Type RA20 VxR fitted with a rotor hosting a 20 microns openings polypropylene basket. The reaction mixture slurry was gradually poured into the basket at room temperature while the centrifuge was gradually spinning to 2000rpm. Once all the liquid has been added the centrifugation process was carried out for 30 minutes at 2000rpm and the liquid was collected at the centrifuge outlet.

The resulting solid collected in the basket and the liquid were analyzed by HPLC following the method described by D.Verraest et al.,in Carbohydrate Research, 271, 101-112 (1995), except for the sodium chloride content which was measured via classical titration with AgNO₃.

The precipitate is characterized by:

| | | |
|---|---|---|
| - | Sodium Chloride: | 86.50 % by weight |
| - | Sodium salt of carboxymethyl inulin: | 5.31 % by weight |
| - | Sodium glycolate | 1.60 % by weight |
| - | Sodium diglycolate | 1.10 % by weight |

The aqueous solution is characterized by:

| | | |
|---|---|---|
| - | Sodium salt of carboxymethyl inulin: | 30.60% by weight |
| - | Degree of carboxymethyl substitution (DS) | 2.47 |
| - | Sodium Chloride: | 6.90 % by weight |
| - | Sodium glycolate: | 10.00% by weight |
| - | Sodium diglycolate: | 6.85 % by weight |
| - | Sodium salt of monochloroacetic acid: | < 0.1 % by weight |

### Example 2.

In a 1 liter round-bottom flask, 38.8 g of monochloroacetic acid sodium salt (SMCA) were dissolved into 95.8 g of water under stirring, while the reaction flask was heated up to a temperature of about 35-40°C. To this solution, 142.0 g of Inulin (Fibruline® instant from Cosucra-Warcoing) were added, under vigorous stirring. A viscous slurry was obtained. The temperature was gradually increased to 70°C. Subsequently a 50% by weight aqueous solution of sodium hydroxide was added to the slurry at an overall rate of 1.9 g per minute during 8 minutes. The reaction medium, thus obtained, has a temperature of about 75°C and a pH value above 10.2.

Then, a simultaneous addition of a 50% by weight aqueous solution of sodium hydroxide, on the one hand, and SMCA on the other hand was carried out in such a way as to maintain the reaction temperature in the range 74 to 78°C and the pH value above 10.2;
Therefore:
- the 50% by weight aqueous solution of sodium hydroxide was added at an overall rate of 2.15 g per minute during 88 minutes (or a total amount of 189.2 g);
- the solid SMCA was added in portions at an overall rate of 3.5 g per minute during 67 minutes (or a total amount of 234.5 g).

After completion of the reactants addition, the dark slurry (total amount of 715.4 g) was maintained at 75°C for a further period of 1 hour under stirring.

Then, the reaction mixture was cooled down, while stirring, to ambient temperature whereupon a precipitate was formed.

The precipitate was separated from the aqueous solution: 715.4 g of the above slurry were poured into a centrifuge whereupon 136.7 g of precipitate and 561.2 g of aqueous solution were obtained.

The precipitate is characterized by:

| | | |
|---|---|---|
| - | Sodium Chloride: | 73.74 % by weight |
| - | Sodium salt of carboxymethyl inulin: | 12.31 % by weight |
| - | Sodium glycolate | 2.90 % by weight |
| - | Sodium diglycolate | 1.40 % by weight |

The aqueous solution is characterized by:

| | | |
|---|---|---|
| - | Sodium salt of carboxymethyl inulin: | 38.30% by weight |
| - | Degree of carboxymethyl substitution (DS) | 2.11 |
| - | Sodium Chloride: | 6.90 % by weight |
| - | Sodium glycolate: | 9.40% by weight |
| - | Sodium diglycolate: | 4.60 % by weight |
| - | Sodium salt of monochloroacetic acid: | <0.1 % by weight |

### Example 3.

In a 1 liter round-bottom flask, 54.3 g of monochloroacetic acid sodium salt (SMCA) were dissolved into 144.0 g of water under stirring, while the reaction flask was heated up ca. 35-40°C. To this solution, 198.8 g of Inulin (Fibruline® instant from Cosucra-Warcoing) were added under vigorous stirring. Temperature was gradually increased up to 70°C. Subsequently, a 50% by weight aqueous solution of sodium hydroxide was added into the Inulin/SMCA mixture at an overall rate 2.5 g per min. during 6 min. leading to a reaction mixture temperature at about 75°C and a pH value above 10.2.

Then, a simultaneous addition of 50% by weight aqueous solution hydroxide and solid SMCA was carried out in such a way as to maintain the reaction temperature in the range 74 to 78°C and the pH value above 10.2;
Therefore:
- the 50% by weight aqueous solution hydroxide was added at an averall rate of about 2.51 g per min. during 68 minutes(or a total amount of 170.7g);
- the solid SMCA was added in portions at an overall rate of 4.03 g per min. during 46 minutes (or a total amount of 185.4 g).

After completion of the reactants addition, the dark slurry (total amount of 768.1 g) was maintained at 75°C for a further period of 1 hour under stirring.

Then, the reaction mixture was cooled down, while stirring, to ambient temperature whereupon a precipitate was formed.

The precipitate was separated from the aqueous solution, by centrifugation of the above slurry, whereupon 92.8 g of precipitate and 660.53g of aqueous solution were obtained.

The precipitate is characterized by:

| | | |
|---|---|---|
| - | Sodium Chloride: | 73.20 % by weight |
| - | Sodium salt of carboxymethyl inulin: | 12.43 % by weight |
| - | Sodium glycolate: | 2.40 % by weight |
| - | Sodium diglycolate: | 1.02 % by weight |

The aqueous solution is characterized by:

| | | |
|---|---|---|
| - | Sodium salt of carboxymethyl inulin: | 36.90% by weight |
| - | Degree of carboxymethyl substitution (DS) | 1. 54 |
| - | Sodium Chloride: | 7.96 % by weight |
| - | Sodium glycolate: | 7.90 % by weight |
| - | Sodium diglycolate: | 2.10 % by weight |
| - | Sodium salt of monochloroacetic acid: | <0.1 % by weight |

**Comparative Example.** (according to a method of the state of the art)

To the dark slurry, of Example 1, 614.4 g of cold water were added, while stirring, to produce 1382.2 g of a homogeneous solution characterized by the following analytical parameters:

| | | |
|---|---|---|
| - | Sodium salt of carboxymethyl inulin: | 15.50 % by weight |
| - | Degree of carboxymethyl substitution (DS): | 2.47 |
| - | Sodium glycolate: | 5.85 % by weight |
| - | Sodium diglycolate: | 3.85 % by weight |
| - | Sodium salt of monochloroacetic acid: | <0.1 % by weight |
| - | Sodium Chloride: | 7.85 % by weight |

This solution is an homogeneous liquid at room temperature.

The comparative example proves that performing a method according to the state of the art, only allows for a homogeneous aqueous solution comprising 15.5 % by weight of alkali metal salt of carboxymethyl inulin, with a degree of carboxymethyl substitution of 2.47. Applying the method according to the present invention allows for a homogeneous aqueous solution comprising 30.6 % by weight of alkali metal salt of carboxymethyl inulin having the same degree of carboxymethyl substitution.

The examples above clearly show that the method of the present invention yields concentrated aqueous solutions (≥ 20% by weight) of alkali metal salt of carboxymethyl inulin characterized by a high degree of carboxymethyl substitution (between 1.2 and 3); the aqueous solutions are further characterized by low concentrations of alkali metal halogenide salt (<10% by weight). The method of the present invention allows for the preparation of said aqueous solutions in an easy and economical way.

It is quite important to notice that the aqueous solutions obtained according to the method of the present invention have a low alkali metal halogenide salt concentration. Surprisingly it is observed that upon cooling the reaction medium, after completion of the carboxymethylation reaction, the precipitate formed almost only comprises alkali metal halogenide salt while the organic compounds, more particularly the alkali metal salt of carboxymethyl inulin, remain dissolved in the water.

## Claims

1. A method for the manufacture of an aqueous solution of alkali metal salt of carboxymethyl fructan, comprising the steps of:
a) reacting, in an aqueous medium, a fructan with an alkali metal salt of a halogenomethylcarboxylate in the presence of an alkaline hydroxide at a temperature comprised between 50°C and 100°C to form a reaction medium;
b) cooling the said reaction medium to a temperature lower than 40°C to form a slurry comprising an alkali metal halogenide precipitate;
c) separating the alkali metal halogenide precipitate from the slurry and collecting an aqueous solution comprising at least 20% by weight of the alkali metal salt of carboxymethyl fructan with a degree of carboxymethyl substitution of at least 1.2.

2. The method according to claim 1 wherein step a) comprises the steps of:
a.1) adding the fructan and the alkaline hydroxide to the aqueous medium while heating to a temperature comprised between 60°C and 100°C, to yield a reaction medium comprising between 25 % and 70% by weight of fructan, expressed in relation to the amount of water, and at a pH in the range comprised between 8 and 13;
a.2) simultaneously gradually adding the alkali metal salt of the halogenomethylcarboxylate and the alkaline hydroxide to yield a molar ratio of the alkali metal salt of halogenomethylcarboxylate to a repetitive monosaccharide unit of the fructan of from 1.0 to 5.0 and to a pH in the range comprised between 8 and 13;
a.3) maintaining the reaction medium, at a temperature comprised between 60°C and 100°C and completing a carboxymethylation reaction.

3. The method according to the claim 1 or 2 wherein the aqueous medium of step a) is water or an aqueous dispersion comprising from 25% to 150% mole, expressed in relation to the molar amount of monosaccharide units in the fructan (100%), of the alkali metal salt of halogenomethylcarboxylate.

4. The method according to any of the preceding claims wherein:
- the halogen of the alkali metal salt of halogenomethylcarboxylate is selected from the group consisting of chlorine, bromine and iodine;
- the alkaline ion is selected from the group consisting of sodium and potassium.

5. The method according to any of the preceding claims wherein the alkali metal salt of halogenomethylcarboxylate of step a) is the sodium salt of monochloroacetic acid or the potassium salt of monochloroacetic acid or a mixture thereof.

6. The method according to any of the preceding claims wherein the aqueous solution obtained from step c) comprises from 20% to 50% by weight and preferably from 25% to 40% by weight of an alkali metal salt of carboxymethyl fructan.

7. The method according to any of the preceding claims wherein the alkali metal salt of carboxymethyl fructan of the aqueous solution obtained from step c) is **characterized by** a degree of carboxymethyl substitution comprised between 1.2 and 3.0.

8. The method according to any of the preceding claims wherein the aqueous solution obtained from step c) comprises less than 10% by weight of alkali metal halogenide salt.

9. The method according to any of the preceding claims wherein step a.1) and a.2) are performed while maintaining the temperature of the reaction medium between 70°C and 90°C and the pH between 9.5 and 11.5.

10. The method according to any of the preceding claims wherein step a.3) is performed while maintaining the temperature of the reaction medium between 70°C and 90°C.

11. The method according to any of the preceding claims wherein the reaction medium of step a) is cooled down in step b) to a temperature comprised between 15°C and 30°C, preferably between 15°C and 25°C.

12. The method according to any of the preceding claims wherein the fructan is inulin.

13. An aqueous solution of an alkali metal salt of carboxymethyl fructan obtained from the method according to any of the previous claims, which comprises at least 20% weight of alkali metal salt of carboxymethyl fructan and wherein the alkali metal salt of carboxymethyl fructan presents a degree of carboxymethyl substitution of at least 1.2.

14. The aqueous solution of an alkali metal salt of carboxymethyl fructan obtained from the method according to any of the previous claims, which comprises between 20% and 50% by weight of an alkali metal salt of carboxymethyl fructan, between 5% and 10% by weight of an alkali metal halogenide salt, between 5% and 15% by weight of an alkali metal glycolate salt and between 1% and 10% by weight of an alkali metal diglycolate salt.

15. The aqueous solution of an alkali metal salt of carboxymethyl fructan according to the claim 13 or 14, wherein the fructan is inulin.

16. Use of the aqueous solution of an alkali metal salt of carboxymethyl fructan according to any of the preceding claims 13 to 15 or obtained from the method according to any of the previous claims 1 to 12, as scale inhibitor, a dispersing agent and/or a metallic cation sequestering agent.
